Europäisches Patentamt

(19) European Patent Office

Office européen des brevets

(11) Publication number: **0 214 243**
**B1**

(12) **EUROPEAN PATENT SPECIFICATION**

(45) Date of publication of patent specification: **18.07.90**

(51) Int. Cl.⁵: **A 61 M 16/00**

(21) Application number: **86901722.8**

(22) Date of filing: **27.02.86**

(86) International application number:
**PCT/US86/00456**

(87) International publication number:
**WO 86/05102 12.09.86 Gazette 86/20**

(54) TRACHEOSTOMY DEVICE.

(30) Priority: **08.03.85 US 709543**

(43) Date of publication of application:
**18.03.87 Bulletin 87/12**

(45) Publication of the grant of the patent:
**18.07.90 Bulletin 90/29**

(84) Designated Contracting States:
**AT BE CH DE FR GB IT LI LU NL SE**

(56) References cited:
US-A-3 137 299   US-A-4 325 366
US-A-3 693 624   US-A-4 416 273
US-A-3 844 290   US-A-4 506 665
US-A-4 029 105   US-A-4 510 933
US-A-4 040 428

"BNGNA Low Resistance Voice Prosthesis",
Advertisement of Bivona Surgical, Inc. 5700
West 23rd Avenue, Gary, Indiana 46406
Blom et al, "Introducing the American V. Mueller
Voice Restoration System", Number

(73) Proprietor: **PASSY & PASSY, INC.**
**4521 Campus Drive Suite 273**
**Irvine, CA 92714 (US)**

(72) Inventor: **MUIR, David A.**
**210 Paseo Picaro**
**Anaheim, CA 92807 (US)**

(74) Representative: **Betten, Jürgen, Dipl.-Ing. et al**
**Patentanwälte Betten & Resch**
**Reichenbachstrasse 19**
**D-8000 München 5 (DE)**

(56) References cited:
BE02108301, 1983, American Hospital Supply
Corporation, 6600 West Touhy Avenue,
Chicago, Illinois, USA 60648.
Blom et al, "Tracheostoma Valve For Post
Laryngectomy Voice Rehabilitation", Ann Otol
Rhinol Laryngol 91: 1982, Pages 576-578
"For All Your Tracheostomy Needs", Publication
of Shiley Incorporated, 1984, Shiley Inc.

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**Description**

In a tracheotomy, an opening is formed in the lower region of the patient's neck into the trachea. When this is done, ambient air can flow directly from the atmosphere to the lungs.

After a tracheotomy, a tracheostomy device is commonly employed to conduct ambient air through the opening in the neck to the trachea. A tracheostomy device typically includes a tube releasably attached at its outer end to the patient and extending through the opening in the neck into the trachea. The tube may be used alone for this purpose, or the tube may be an inner cannula which is received within an outer cannula.

A patient with a tracheotomy tends to inhale and exhale through the tracheostomy device. The larynx or voice box is located above the tracheostomy device and so, when the patient exhales through the device, air flow through the larynx is minimal. Without air flow through the larynx and out through the mouth, speech is difficult or impossible. To aid speech, the patient may place his finger over the opening in the tracheostomy device whenever he wishes to speak so that he will then exhale through the larynx and mouth to make speech possible. This technique is inconvenient because it requires manual manipulation to close off the tracheostomy device during exhalation to enable speech and opening the device to enable inhalation. This requires the patient to coordinate manual and breathing functions and cannot be done if the patient does not have adequate use of at least one arm and hand.

A device according to the pre-characterizing part of claim 1 is known from US—A—3,137,299. In this prior art airflow is not totally blocked during all of the exhalation phase and in the neutral phase since the flexible resilient valve element is not positively biased against the valve seat in the absence of a force of exhalation and since there is an air leakage between the cylindrical cup 5 of the tube and the cup 12 of the valve.

A number of undesirable effects result from less than total blockage of airflow through a tracheostomy valve during all or part of the exhalation phase of the respiration cycle. The passage of moisture-laden breath through the tracheostomy tube to the ambient atmosphere tends to produce deposits of moisture and mucus on the walls of the valve housing. Such deposits tend to increase friction, making the valve more difficult to open during inhalation. They may also act as a culture medium for pathogens, such as pseudamonas bacteria. Further, such valves tend to click and produce vibration in use, particularly at the beginning of the exhalation cycle, which disturbs the patient. Finally, the speech of users of such valves is not normal and is frequently unintelligible.

This invention solves these problems by providing a device according to claim 1. The one-way valve of the device automatically permits the patient to inhale through the tube and blocks exhalation through the tube.

The tracheostomy tube has a passage with an inlet adapted to be exposed to ambient or atmospheric air and an outlet adapted to be in the patient's trachea so that the tube can conduct ambient air to the trachea. The one-way valve permits air flow through the passage from the inlet to the outlet and completely blocks the flow of exhalation air through the passage from the outlet through the inlet to the atmosphere to cause adequate air flow along a path which includes the larynx and the nose or mouth to materially facilitate speech.

The tube has opposite ends, and the inlet is preferably substantially at one of the ends. Although the one-way valve can be carried at different locations by the tube, preferably, the one-way valve is mounted on the inlet end of the tube. In a preferred construction, the mounting means removably mounts the one-way valve on the inlet end of the tube. Consequently, the valve can be removed by the patient or an attendant if it is desired to exhale through the tube.

The tracheostomy tube can be an inner cannula adapted to be removably recieved in an outer cannula. Alternatively, the tracheostomy tube may be an outer cannula which may or may not have an inner cannula.

The invention, together with additional features and advantages thereof, may best be understood by reference to the following description taken in connection with the accompanying illustrative drawing.

Fig. 1 is an exploded isometric view of a portion of one form of tracheostomy device constructed in accordance with the teachings of this invention.

Fig. 2 is a view taken generally along line 2—2 of Fig. 1.

Fig. 3 is a sectional view taken generally along line 3—3 of Fig. 2 with the components of the tracheostomy device assembled.

Fig. 4 is an exploded isometric view of a second form of tracheostomy device constructed in accordance with the teachings of this invention.

Fig. 5 is a somewhat schematic view illustrating one way in which the tracheostomy device can be used.

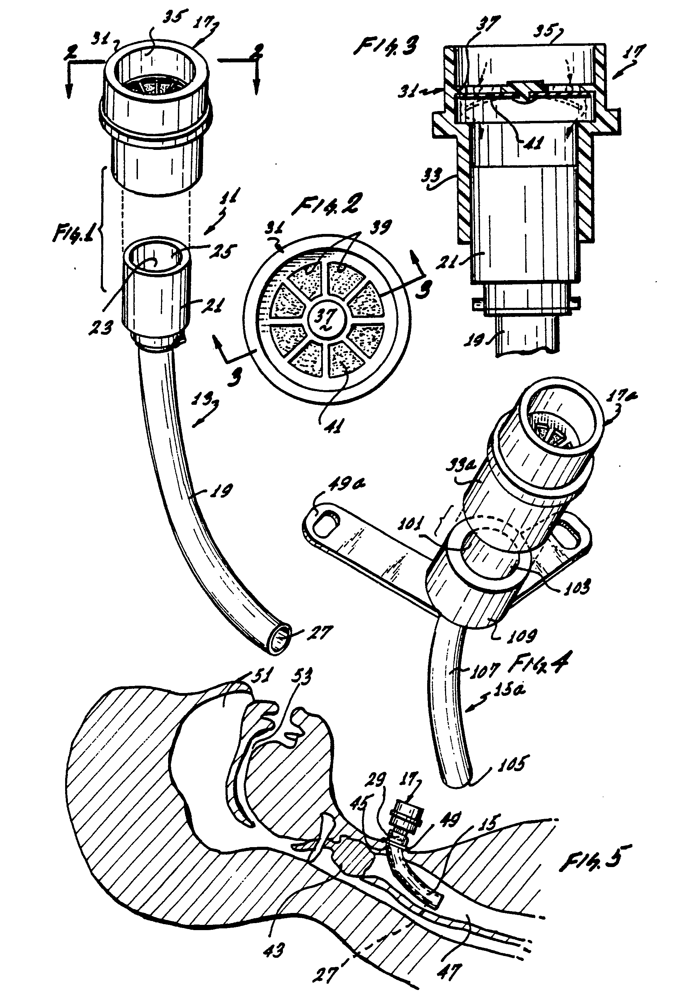

Figs. 1—3 and 5 show a tracheostomy device 11 which generally comprises a tracheostomy tube in the form of an inner cannula 13, an outer cannula 15 (Fig. 5) and a one-way valve 17. The cannulas 13 and 15 may be conventional. As such, the inner cannula 13 includes a plastic tube 19 and a coupling 21 rotatably mounted on one end of the tube 19. The inner cannula 13 has a passage 23 extending completely through it from an inlet 25 to an outlet 27.

The outer cannula 15 is also in the form of a tube which is sized to receive the tube 19 of the inner cannula 13 therein. The outer cannula 15 has a fixed coupling 29 at its inlet end for at least partially recieving and cooperatively locking with the coupling 21 to lock the inner cannula 13 to the outer cannula 15 in a conventional manner.

Although the one-way valve 17 can be of various different constructions, in the embodiment illustrated, it includes a tubular valve housing 31 having a coupling section 33 which is adapted to slidably receive at least a portion of the coupling 21 with an interference fit so that the one-way valve is retained on the inner cannula 13 by friction. One or both of the engaging surfaces of the coupling 21 and the coupling section 33 can be tapered to facilitate such a friction fit, if desired.

The end of the housing 31 remote from the coupling section 33 terminates in an opening 35. A transverse wall 37 extends across the housing 31 intermediate the opening 35 and the coupling section 33. The wall 37 has a plurality of ports 39 extending through the wall, and a flexible resilient valve element 41 is suitably attached to a central region of the transverse wall 37 and extends radially outwardly to fully cover each of the ports 39.

This invention is particularly adapted for use with a patient having a larynx 43 (Fig. 5) and a tracheotomy which leaves an opening 45 in the patient's neck extending from the atmosphere to the trachea 47. The outer cannula is inserted through the opening 45 to the trachea 47 and is retained on the patient's neck in any suitable manner, such as by wrapping a cord or flexible element (not shown) about the neck and affixing it to a cuff 49 which is carried by, and forms a portion of, the outer cannula 15. The inner cannula 13 is inserted into the outer cannula 15 and is affixed thereto in a conventional manner by rotation of the coupling 21 to attach the coupling 21 to the coupling 29. When affixed in this fashion, the outlet 27 terminates within the outer cannula 15 as shown in Fig. 5.

The one-way valve 17 is removably mounted on the inlet end of the inner cannula 13 by virtue of the friction fit between the coupling section 33 and the coupling 21 as shown in Fig. 3. When installed in this fashion, the one-way valve 17 permits air flow through the passage 23 from the atmosphere to the trachea, and it completely blocks air flow through the passage from the trachea through the passage 23 to the atmosphere. Accordingly, when the patient inhales, ambient or atmospheric air is supplied through the one-way valve 17 and the passage 23 to the trachea 47. However, when the patient exhales, there is no air flow through the passage 23 to the atmosphere. Rather, exhalation air is forced along a path which includes the larynx 43 and the nose 51 and/or the mouth 53 to the atmosphere. Because the exhalation air flows over the larynx 43 and out the mouth 53, the patient is automatically enabled to speak. The one-way valve 17 can be manually removed from the inner cannula 13 when desired.

Fig. 4 shows the use of a one-way valve 17a with a tracheostomy tube in the form of an outer cannula 15a. Portions of the embodiment of Fig. 4 corresponding with portions of the embodiment of Figs. 1—3 and 5 are designated by corresponding reference numerals followed by the letter "a".

The outer cannula 15a is of the type which does not utilize an inner cannula. Thus, the outer cannula 15a has a passage 101 extending through it from an inlet 103 to an outlet 105. The outer cannula 15a also has a cuff 49a for use in removably attaching the outer cannula 15a to the neck of a patient. The outer cannula 15a also includes a tube 107 and an enlarged head 109.

The one-way valve 17a may be identical to the one-way valve 17, except that the coupling section 33a is sized to frictionally receive the head 109 to removably mount the one-way valve on the outer cannula 15a. The embodiment of Fig. 4 may be used in the same manner as described above.

The one-way valves 17 and 17a can be used with many different tracheostomy tubes, and the tracheostomy tubes described herein are merely illustrative. Also, the one-way valve can be of various different constructions which will carry out the basic functions described herein.

**Claims**

1. A tracheostomy device comprising:
   a tracheostomy tube (19) having an outer end portion adapted to be at least partially received in a patient's trachea (47), said tracheostomy tube having a passage (23) extending therethrough;
   said passage having an inlet (25) at said outer end portion adapted to be exposed to ambient air and an outlet (27) adapted to be in the patient's trachea whereby the tube can conduct ambient air to the patient's trachea;
   a one-way valve (17) including a tubular valve housing (31) slidably cooperable with the outer end portion of the tube to removably mount the valve housing on the outer end portion of the tube whereby the valve housing can be installed on and removed from the outer end portion of the tube; and
   said one-way valve (17) including a valve carried by said tubular valve housing for permitting air flow through said passage from said inlet to said outlet when said patient inhales and for blocking air flow through said passage from said outlet through said inlet to the atmosphere at least with the force of exhalation to cause air flow along a path which includes the larynx and the nose or mouth of said patient, said valve including a flexible resilient valve element (41), characterized in that:
   —said tracheostomy device is a speaking device;
   —said tubular valve housing (31) forms a friction fit with said outer end portion of said tube (12); and
   —said valve element (41) completely blocks air flow at all times except when said patient inhales.

2. A device as defined in claim 1 including an outer cannula (15) adapted to extend from ambient air into the patient's trachea and said tube is an inner cannula (13) removably received within the outer cannula.

3. A device as defined in claim 1 wherein the valve housing (31) and the outer end of the tube (19) have engaging surfaces and at least one of the engaging surfaces is tapered.

4. A device as defined in claim 1 wherein said valve (17) includes a transverse wall (37) extending across the valve housing (31) intermediate the ends thereof whereby the transverse wall is recessed inwardly from both ends of the valve housing, said transverse wall having a port (39) therein and said flexible resilient valve element (41) being coupled to the transverse wall.

5. A device as defined in claim 1 wherein said inner end of said tube includes a coupling (31) and said valve housing (31) includes a coupling section (33) which slidably receives said coupling with said friction fit.

6. A device as defined in claim 1 wherein the valve housing (31) and the outer end of the tube (19) have engaging surfaces and at least one of the engaging surfaces is tapered.

**Patentansprüche**

1. Trachealkanülengerät mit:

einem Trachealrohr (19) mit einem äußeren Endabschnitt, der zumindest teilweise in der Luftröhre (47) eines Patienten aufnehmbar ist, wobei das Trachealrohr einen Durchgang (23) aufweist, der sich durch dieses hindurcherstreckt;

wobei der Durchgang einen Eingang (25) am äußeren Endabschnitt aufweist, der der Umgebungsluft aussetzbar ist und einen Ausgang (27), der dazu ausgebildet ist, in der Luftröhre des Patienten angeordnet zu sein;

einem Einwegeventil (17) mit einem rohrförmigen Ventilgehäuse (31), welches gleitbar mit dem äußeren Endabschnitt des Rohres zusammenwirkt, um das Ventilgehäuse am äußeren Endabschnitt des Rohres entfernbar zu tragen, wodurch das Ventilgehäuse am äußeren Endabschnitt des Rohres befestigbar ist und von diesem wieder entfernbar ist;

und wobei das Einwegeventil (17) ein Ventil umfaßt, welches vom rohrförmigen Ventilgehäuse getragen ist, um eine Luftströmung durch den Durchgang vom Eingang zum Ausgang zu ermöglichen, wenn der Patient einatmet, und um eine Luftströmung durch den Durchgang vom Ausgang zum Eingang zu der Umgebungsluft zumindest mit der Kraft der Ausatmung zu blokkieren, um zu bewirken, daß die Luft entlang einer Bahn strömt, welche den Kehlkopf und die Nase oder den Mund des Patienten umfaßt, wobei das Ventil ein flexibles federndes Ventilelement (41) umfaßt, dadurch gekennzeichnet, daß

—das Trachealkanülengerät ein Sprechgerät ist,

—das rohrförmige Ventilgehäuse (31) einen Reibungssitz mit dem äußeren Endabschnitt des Rohres (12) bildet, und

—das Ventilelement (41) eine Luftströmung zu jeder Zeit vollständig blockiert mit Ausnahme dann, wenn der Patient einatmet.

2. Gerät nach Anspruch 1 mit einer äußeren Kanüle (15), welche dazu ausgebildet ist, sich von der Umgebungsluft in die Luftröhre das Patienten zu erstrecken, wobei das Rohr eine innere Kanüle (13) ist, die in der äußeren Kanüle entfernbar aufgenommen ist.

3. Gerät nach Anspruch 1, wobei das Ventilgehäuse (31) und das äußere Ende des Rohres (19) miteinander in Eingriff kommende Oberflächen aufweisen und zumindest eine der miteinander in Eingriff kommenden Oberflächen geneigt ist.

4. Gerät nach Anspruch 1, wobei das Ventil (17) eine Querwandung (37) umfaßt, welche sich über das Ventilgehäuse (31) zwischen deren Enden erstreckt, wodurch die Querwandung von beiden Enden des Ventilgehäuses zurückspringt, wobei die Querwandung eine Öffnung (39) aufweist und das flexible federnde Ventilelement (41) mit der Querwandung gekoppelt ist.

5. Gerät nach Anspruch 1, wobie das innere Ende des Rohres eines Kupplung (31) umfaßt und das Ventilgehäuse (31) einen Kupplungsabschnitt (33) umfaßt, welcher die Kupplung mit dem Reibungssitzt gleitbar aufnimmt.

6. Gerät nach Anspruch 1, wobei das Ventilgehäuse (31) und das äußere Ende des Rohres (19) miteinander in Eingriff kommende Oberflächen aufweisen und zumindest eine der miteinander in Eingriff kommenden Oberflächen geneigt ist.

**Revendications**

1. Un appareil trachéostomique comprenant:

un tube trachéostomique (19) présentant une partie d'extrémité extérieure adaptée pour être au moins partiellement placée dans la trachée (47) d'un patient, ledit tube trachéostomique présentant un passage intérieur (23);

ledit passage comportant une entrée (25) sur ladite partie d'extrémité extérieure, adaptée pour être exposée à l'air ambiant, et une sortie (27) adaptée pour être placée dans la trachée du patient, de manière à ce que le tube puisse amener l'air ambiant à la trachée du patient; une valve unidirectionnelle (17) comprenant un carter tubulaire (31) de valve, coopérant par coulissement avec la partie d'extrémité extérieure du tube afin de monter de manière amovible le carter de valve sur la partie d'extrémité extérieure du tube, de manière à ce que le carter de valve puisse être installé sur la partie d'extrémité extérieure du tube et enlevé de cette partie; et

ladite valve unidirectionnelle (17) comprenant une valve portée par ledit carter tubulaire de valve, afin de permettre un écoulement d'air à travers ledit passage, en allant de ladite entrée à ladite sortie, lorsque ledit patient inhale et afin de bloquer l'écoulement d'air à travers ledit passage, en allant de ladite sortie, par ladite entrée, à l'atmosphère, au moins avec la force d'exhalaison pour provoquer un écoulement d'air le long d'un trajet qui comprend le larynx et le nez ou la bouche dudit patient, ladite valve comprenant un organe de valve élastique flexible (41), caractérisé en ce que:

—ledit dispositif trachéostomique est un dispositif porte-voix;

—ledit carter tubulaire de valve (31) forme un montage à friction avec ladite partie d'extrémité extérieure dudit tube (12); et

—ledit organe de valve (41) bloque complètement l'écoulement d'air en permanence, sauf lorsque ledit patient inhale.

2. Un appareil tel que défini à la revendication 1, comprenant une canule extérieure (15) adaptée pour s'étendre, depuis l'air ambiant jusque dans la trachée du patient et ledit tube étant une canule intérieure (13) logée de manière amovible à l'intérieur de la canule extérieure.

3. Un appareil tel que défini à la revendication 1, dans lequel le carter de valve (31) et l'extrémité extérieure du tube (19) comportant des surfaces de contact et qu'au moins l'une des surfaces de contact est conique.

4. Un appareil tel que défini à la revendication 1, dans lequel ladite valve (17) comprend une paroi transversale (37) s'étendant dans le carter de valve (31), entre ses extrémités, de manière que la paroi transversale soit creusée vers l'intérieur depuis les eux extrémités du carter de valve, ladite paroi transversale présentant en son sein un orifice (39) et ledit organe flexible élastique (41) de valve étant couplé à la paroi transversale.

5. Un appareil tel que défini à la revendication 1, dans lequel ladite extrémité intérieure dudit tube comprend un accouplement (21) et ledit carter (31) de valve comprend une section de couplage (33) qui reçoit de manière coulissante ledit accouplement avec un ajustement à friction.

6. Un appareil tel que défini à la revendication 1, dans lequel ledit carter de valve (31) et l'extrémité extérieure du tube (19) comportent des surfaces de contact et qu'au moins l'une des surfaces de contact est conique.

EP 0 214 243 B1

FIG. 1

FIG. 2

FIG. 3

FIG. 4

FIG. 5